Europäisches Patentamt

European Patent Office

Office européen des brevets

(19)

(11) Publication number: **0 569 042 A1**

## EUROPEAN PATENT APPLICATION

(12)

(21) Application number: **93107660.8**

(22) Date of filing: **12.03.87**

(51) Int. Cl.5: **A61K 37/02**

This application was filed on 11 - 05 - 1993 as a divisional application to the application mentioned under INID code 60.

(30) Priority: **14.03.86 JP 57885/86**
**20.06.86 JP 145830/86**
**09.07.86 JP 161184/86**
**27.08.86 JP 200324/86**

(43) Date of publication of application:
**10.11.93 Bulletin 93/45**

(60) Publication number of the earlier application in accordance with Art.76 EPC: **0 237 967**

(84) Designated Contracting States:
**CH DE FR GB IT LI NL SE**

(71) Applicant: **OTSUKA PHARMACEUTICAL CO., LTD.**
**Sawanotsuru Bldg., 1-2, Hiranomachi 2-chome**
**Chuo-ku, Osaka 541(JP)**

(72) Inventor: **Nakai, Satoru**
**674, Aza-Minamikawamuko,**
**Hiroshima**
**Matsushige-cho, Itano-gun,**
**Tokushima-ken(JP)**
Inventor: **Kaneta, Mayumi**
**1-81, Higashinakasu,**
**Nakamura**
**Kitajima-cho, Itano-gun, Tokushima-ken(JP)**
Inventor: **Kikumoto, Yoshikazu,**
**Haitsu-Datemusume B-302**
**57-1, Aza-Hachigami,**

**Sasakino,**
**Matsushige-cho**
**Itano-gun, Tokushima-ken(JP)**
Inventor: **Hong, Yeong-Man**
**Sanraunjihausu 105,**
**129-4, Miyanonishi,**
**Kitahama**
**Muya-cho, Naruto-shi, Tokushima-ken(JP)**
Inventor: **Kawai, Kazuyoshi, Haitsu Soreiyu 503**
**130-2, Aza-Mitsuhokaitaku,**
**Mitsuho,**
**Matsushige-cho**
**Itano-gun, Tokushima-ken(JP)**
Inventor: **Takegata, Setsuko**
**1090-18, Kagasuno,**
**Kawauchi-cho**
**Tokushima-shi, Tokushima-ken(JP)**
Inventor: **Ishii, Kiyoshi**
**39-46, Aza-Sakafuji,**
**Sumiyoshi**
**Aizumi-cho, Itano-gun, Tokushima-ken(JP)**
Inventor: **Yanagihara, Yasuo**
**891-6, Oomatsu,**
**Kawachi-cho**
**Tokushima-shi, Tokushima-ken(JP)**
Inventor: **Hirai, Yoshikatsu**
**5-49, Aza-Ekogawa,**
**Shinkirai**
**Kitajima-cho, Itano-gun, Tokushima-ken(JP)**

(74) Representative: **VOSSIUS & PARTNER**
**Postfach 86 07 67**
**D-81634 München (DE)**

(54) **Medical uses of IL-1 beta.**

(57) The invention relates to an interleukin $1\beta$ (IL-$1\beta$), a gene coding for said protein and novel medicinal uses of homogeneous IL-$1\beta$.

EP 0 569 042 A1

EP 0 569 042 A1

The present invention relates to the medical use of IL-1$\beta$.

The Second International Lymphokine Workshop decided to adopt a unified name, interleukin-1 (IL-1), for the physiologically active substances which had been referred to as lymphocyte activating factor (LAF), mitogenic protein, helper peak-1, T-cell replacing factor III (TRF-III), T-cell replacing factory M$\phi$ (TRFM), B-cell activating factor, B-cell differation factor, etc. (Cellular Immunol., 48, 433-436 (1979)). This decision is based on the reason that these physiologically active substances can not be distinguished from one another as different substances but are expressed variously merely with reference to physiological activities as interpreted from different angles.

Further it is reported that IL-1 activates T lymphocytes and B lymphocytes, has activity to promote production of interleukin-2 and antibodies, acts on liver tissues to promote protein synthesis and possesses activity to promote production of prostaglandins (see Reviews of Infectious Disease, Vol. 6, No.1, 51-59 (1984), New England J. of Med., 311, 1413 (1984), etc.).

Whereas IL-1 itself still remains to be clarified as a substance, it is only recently that reports are made on the presence of two different genes coding for polypeptides having LAF activity or precursor thereof (Proc. Natl. Acad. Sci., Vol. 81, 7907-7911 (1984), Nature, Vol. 315, 641 (1985), Nucleic Acid Research, Vol. 13 (16), 5869 (1985)). These reports mention "IL-1$\alpha$" having a sequence of 159 amino acids and "IL-1$\beta$" comprising 153 amino acids which are postulated from the base sequences of the two genes.

Nevertheless, research has yet to be made to clarify the relation between said activities assayed using a conditioned medium or so called partially purified IL-1 and the polypeptide postulated from the above base sequences.

Known IL-1 as a physiologically active substance is also thought to be identical with an endogeneous pyrogen (EP) and is known to exhibit a pyrogenic property (see Nature, 304, 449 (1983); Cell Immunol., 63, 164 (1981); J. Exp. Med., 150, 709 (1979); J. Immunol., 130, (6) 2708 (1983); same, 132 (3) 1311 (1984); etc.). This indicates that IL-1, if available as a uniform substance, to say nothing of physiologically active conventional IL-1, is difficult to use for medical applications.

Another object of the invention is to provide a gene coding for IL-1$\beta$ and a process for preparing IL-1$\beta$.

Another object of the invention is to provide novel medicinal uses for homogeneous IL-1$\beta$ itself.

The above objects of the invention will become apparent from the following description.

The amino acid sequence of interleukin-1$\beta$ is represented by the formula (A).

2

Formula (A)

```
                              5                          10
        Ala - Pro - Val - Arg - Ser - Leu - Asn - Cys - Thr - Leu -

                              15                         20
        Arg - Asp - Ser - Gln - Gln - Lys - Ser - Leu - Val - Met-

                              25                         30
        Ser - Gly - Pro - Tyr - Glu - Leu - Lys - Ala - Leu - His-

                              35                         40
        Leu - Gln - Gly - Gln - Asp - Met - Glu - Gln - Gln - Val-

                              45                         50
        Val - Phe - Ser - Met - Ser - Phe - Val - Gln - Gly - Glu-

                              55                         60
        Glu - Ser - Asn - Asp - Lys - Ile - Pro - Val - Ala - Leu-

                              65                         70
        Gly - Leu - Lys - Glu - Lys - Asn - Leu - Tyr - Leu - Ser-

                              75                         80
        Cys - Val - Leu - Lys - Asp - Asp - Lys - Pro - Thr - Leu-

                              85                         90
        Gln - Leu - Glu - Ser - Val - Asp - Pro - Lys - Asn - Tyr-

                              95                         100
        Pro - Lys - Lys - Lys - Met - Glu - Lys - Arg - Phe - Val-

                              105                        110
        Phe - Asn - Lys - Ile - Glu - Ile - Asn - Asn - Lys - Leu-

                              115                        120
        Glu - Phe - Glu - Ser - Ala - Gln - Phe - Pro - Asn - Trp-

                              125                        130
        Tyr - Ile - Ser - Thr - Ser - Gln - Ala - Glu - Asn - Met-

                              135                        140
        Pro - Val - Phe - Leu - Gly - Gly - Thr - Lys - Gly - Gly-

                              145                        150
        Gln - Asp - Ile - Thr - Asp - Phe - Thr - Met - Gln - Phe-

        Val - Ser - Ser
```

Amino acids are herein referred to by symbols according to the nomenclature or rules recommended by IUPAC-IUB or symbols conventionally in the art.

The homogeneous IL-1$\beta$ obtained in accordance with the invention has for example, physiological activities such as LAF activity, activity to inhibit growth of tumor cells (GIF activity), i.e. activity to specifically inhibit growth of tumor cells, activity to promote production of various cytokines such as colony stimulating factor (CSF), interferon (IFN), interleukin-2 (IL-2) and interleukin-3 (IL-3), i.e. activity for example on human cells to greatly promote production of such cytokines, anti-inflammatory activity, activity for example to effectively inhibit progress of arthritis when administered to model animals with arthritis, and activity to prevent radiation injury, i.e. activity to prevent the possible living body disorders or serious side effects that would result from systemic radiation irradiation during bone marrow transplant, radiation irradiation for treatment of cancers and radiation accident. Accordingly, the homogeneous IL-1$\beta$ of the invention is very useful as immune system stimulants, for example, for promoting production of antibodies and enhancing the effect of vaccines, antitumor agents, agents for promoting production of cytokines such as CSF, IFN, IL-2 and IL-3, anti-inflammatory agents, agents for preventing radiation sickness and other like medicinal agents.

The novel finding that the homogeneous IL-1$\beta$ exhibits remarkable effect in treating an inflammation such as arthritis is surprising in view of the fact that IL-1 has been heretofore thought to induce an inflammation indirectly by acting as a mediator.

Besides the above-mentioned medicinal uses, the IL-1$\beta$ of the present invention is very useful, for example, for preparing various useful cytokines in vitro from cell line, etc. because of their activity to promote production of cytokines. Attention has been directed to the preparation in natural-type cytokines, especially those which are glyco-proteins. The present derivative makes it possible to obtain useful cytokines efficiently in large quantities when used as a cytokine production inducting agent.

The IL-1$\beta$ of the present invention can be prepared, for example, by gene engineering techniques using a gene coding for the specific polypeptide of the invention, i.e., by incorporating the gene into a microorganism vector to effect replication, transcription and translation within the cell of the microorganism. This process is advantageous in that it is amenable to mass production.

Although the gene to be used in this process can be totally synthesized through chemical synthesis of nucleic acids by a usual method, for example, by the phosphite triester method (Nature, 310, 105 (1984)) or the like, it is convenient to utilize the gene coding for IL-1$\beta$ or a precursor thereof. By a conventional method involving the above chemical synthesis, the gene is modified to a sequence of nucleic acids coding for the foregoing specific amino acid sequence, whereby the desired gene can be prepared easily.

The gene coding for IL-1$\beta$ or a precursor thereof is already known. We obtained the gene coding for IL-1$\beta$ as disclosed in our copending application No. 85116344.4 and succeeded in preparing IL-1$\beta$ by gene engineering techniques using this gene. The series of gene engineering techniques employed will be described in the reference examples to follow.

The above-mentioned modified sequence of nucleic acids (bases) is prepared also by a known procedure, which executed according to the amino acid sequence of the desired polypeptide (Molecular Cloning Cold Spring Harbor Laboratory (1982)).

For example, cleavage, ligation, phosphorylation, etc. of DNA can be carried out by usual methods including treatment with enzymes such as restriction enzymes, DNA ligase, polynucleotidekinase and DNA polymerase, which are readily available as commercial products. The isolation and purification of the gene and nucleic acids included in these methods are conducted also in the usual manner, for example, by agarose gel electrophoresis. As will be described partially later, the gene obtained is replicated using a usual vector. The DNA fragment coding for the desired amino acid sequence and synthetic linkers can be prepared also easily by the above-mentioned chemical synthesis. The codon corresponding to the desired amino acid and to be used in the above methods is known and is selected as desired. A usual method may be used for this purpose, for example, in view of the frequency of use of the codon of the host to be used (Nucl. Acids Res., 9, 43-73 (1981)). Further for the modification of the codon in the nucleic acid sequence concerned, for example, site-specific mutagenesis (Proc. Natl. Acad. Sci., 81, 5662-5666 (1984)) can be resorted to as usually done which employs a primer comprising a synthetic oligonucleotide coding for the desired modified sequence about 15-30 mer.

The desired gene obtained by the foregoing process can be checked for its base sequence, for example, by the Maxam-Gilbert chemical modification method (Meth. Enzym., 65, 499-560 (1980)) or by the dideoxynucleotide chain termination method using M13 Phage (Messing, J. and Vieira, J., Gene, 19, 269-276 (1982)).

While the above process and procedures therefor will be described in the reference examples and examples to follow, the process is not specifically limited; any process already known in the art may be used.

Thus, the present invention also provides a novel gene coding for a polypeptide having the above-specified amino acid sequence. (The gene will hereinafter be referred to as the "present gene.")

The polypeptide of the present invention can be prepared by usual known gene recombination techniques using the present gene. More specifically, it is produced by preparing a recombinant DNA which can express the present gene in host cells, transforming the DNA into the host cell and incubating the transformant.

Useful host cells can be either eucaryotic or procaryotic cells. The eucaryotic cells include cells of vertebrate animals, yeasts, etc. Generally used as cells of vertebrate animals are, for example, COS cells which are cells of monkey (Y. Gluzman, Cell, 23, 175-182 (1981)), dihydrofolate reductase defective strain of Chinese hamster ovary cell (G. Urlaub and L.A., Chasin, Proc. Natl. Acad. Sci., U.S.A., 77, 4216-4220 (1980)), etc., while useful cells are not limited to these cells. Useful expression vectors of vertebrate cells

4

are those having a promotor positioned upstream of the gene to be expressed, RNA splicing sites, polyadenylation site, transcription termination sequence, etc. These vectors may further have a replication origin when required. Examples of useful expression vectors include pSV2dhfr having an initial promotor of SV40 (S. Subramani, R. Mulligan and P. Berg, Mol. Cell. Biol., 1(9), 854-864), which is not limitative.

Yeasts are widely used as eucaryotic micro-organisms, among which those of the genus Saccharomyces are generally usable. Examples of popular expression vectors of yeasts and like eucaryotic microorganisms include pAM82 having a promotor for acid phosphatase gene (A. Miyanohara et al., Proc. Natl. Acad. Sci., U.S.A., 80, 1-5 (1983), etc.

E. coli and Bacillus subtilis are generally used as procaryotic hosts. The present invention employs, for example plasmid vectors capable of replication in the host. To express the gene in the vector, expression plasmids can be used which have a promotor and SD (Shine-Dalgarno) base sequence at the upstream of the gene and ATG required for initiating protein synthesis. Widely used as host E. coli is E. coli K12 strain. pBR322 is a vector which is generally used. However, these are not limitative, and various known strains and vectors are usable. Examples of promotors usable are tryptophan promotor, $P_L$ promotor, lac promotor, lpp promotor, etc. The gene can be expressed with use of any of these promotors.

To describe the procedure with reference to the case wherein tryptophan promotor is used, vector pTM1 (Fumio Imamoto, Taishya, Vol. 22, 289 (1985)) having tryptophan promotor and SD sequence is used as an expression vector. A gene coding for a desired polypeptide of this invention and having ATG when required is linked to the site of restriction enzyme ClaI which is present downstream from the SD sequence.

Incidentally, not only the direct expression system but a fusion protein expression system is also usable which employs, for example, β-galactosidase, β-lactamase or the like.

The expression vector thus obtained is introduced into host cells and thereby transformed by usual methods. For example, cells chiefly in the logarithmic growth phase are collected, treated with $CaCl_2$ and thereby made to readily accept DNA, whereupon the vector is introduced into the cell. With this method, $MgCl_2$ or RbCl can be made present conjointly with the vector so as to achieve an improved transformation efficiency, as is generally known. The cell can be converted to spheroplast or protoplast before transformation.

The desired transformant thus obtained can be incubated in the usual manner, whereby the desired polypeptide is produced and accumulated. The medium for the incubation may be any of those generally used for incubating cells, such as L medium, E medium, M9 medium, etc. Various carbon sources, nitrogen sources, inorganic salts, vitamins, etc. which are usually known can be admixed with these media. When the tryptophan promotor is used, M9 minimum medium, for example, is usable which has admixed therewith Casamino acid for effecting the action of the promotor. A chemical, such as indoleacrylic acid, for enhancing the action of tryptophan promotor can be added to the medium at a suitable stage of incubation.

The desired polypeptide of the present invention can be isolated from the resulting culture and purified by usual methods. It is desirable to extract the polypeptide from the host under a mild condition as by osmotic shock in order to maintained the higher-order structure thereof.

The above isolation or purification method is conducted substantially by the same method as usually used for separating a protein-like substance from such a biological substance. For example, various procedures are usable utilizing the physical or chemical properties of the desired polypeptide. (See for example, "Biological Data Book II," pp. 1175-1259, 1st edition, 1st print, June 23, 1980, published by Kabushiki Kaisha Tokyo Kagakudojin.) Examples of useful procedures are treatment with use of a usual protein precipitating agent, ultra-filtration, molecular sieve chromatography (gel filtration), liquid chromatography, centrifugation, electrophoresis, affinity chromatography, dialysis, and combinations of such procedures.

The desired polypeptide is separated from the supernatant as partially purified This partial purification is carried out, for example, by a treatment using as a protein precipitating agent an organic solvent such as acetone, methanol, ethanol, propanol or dimethylformamide (DMF), or an acidic reagent such as acetic acid, perchloric acid (PCA) or trichloroacetic acid (TCA), a treatment using a salting-out agent such as ammonium sulfate, sodium sulfate or sodium phosphate and/or ultrafiltration using a dialysis membrane, flat membrane, hollow fiber membrane or the like. These treatments are conducted in the same manner as usually done under usual conditions.

The roughly purified product thus obtained is then subjected to gel filtration, whereby a fraction exhibiting the activity of the desired substance is collected. Useful gel filtration agents are not limited specifically. Such agents include those made of dextran gel, polyacrylamide gel, agarose gel, polyacrylamide-agarose gel, cellulose or the like. Examples of useful agents commercially available are Sephadex G type, Sephadex LH type, Sepharose type, Sephacryl type (all products of Pharmacia), Cellofine (Chisso Corporation), Biogel P type, Biogel A type (both product of Bio-Rad Lab), Ultro gel-C (LKB), TSK-G type (product of Toyo Soda Mfg. Co., Ltd.), etc.

The polypeptide of the present invention can be isolated from the fraction as a homogeneous substance, for example, by subjecting the fraction to affinity chromatography with use of a hydroxyapatite column, ion exchange column chromatography as of the DEAE, CM or SP method, chromatofocusing method, reverse-phase high-performance liquid chromatography or the like, or to a combination of such methods.

The chromatofocusing method can be carried out by various known procedures. Usable as the column is, for example, PBE94 (Pharmacia Fine Chemicals) or the like, as the starting buffer, for example, imidazole-hydrochloric acid or the like, and the eluent, for example, the mixture of Polybuffer 74 (Pharmacia Fine Chemicals) and hydrochloric acid (pH 4.0) or the like.

The reverse-phase high-performance liquid chromatography can be conducted, for example, with $C_4$ Hi-Pore reverse-phase HPLC column (Bio-Rad Laboratories) or the like, using acetonitrile, trifluoroacetic acid (TFA), water of the like, or a mixture of such solvents as the eluent.

In this way, the IL-1$\beta$ (polypeptide) of the present invention can be obtained upon isolation. The gene coding for IL-1$\beta$ affords IL-1$\beta$ through a similar gene recombination procedure.

The polypeptide of the invention, which has outstanding pharmacological activities as already stated, can be formulated into useful preparations for the aforementioned medicinal uses. Examples of such medicinal preparations include immunostimulators for producing antibodies, enhancing the effect of vaccines and curing immunodeficiency, antitumor agents, cytokine production promotors, anti-inflammatory agents, agents for preventing or curing radiation injury, agents for preventing or curing opportunistic infections, etc. These medicinal preparations are formulated usually in the form of pharmaceutical compositions comprising a pharmacologically effective amount of IL-1$\beta$ and a suitable carrier. Examples of useful pharmaceutical carriers include excipients and diluents such as filler, extender, binder, wetting agent, disintegrator, surfactant, etc. which are generally used for preparing pharmaceuticals of the desired form to be used. The form of the pharmaceutical compositions is not specifically limited insofar as they effectively contain the present or IL-1$\beta$ but can be, for example, in the form of tablets, power, granules, pellets or like solid preparation. Usually, however, it is suitable that the composition be in the form of a solution, suspension, emulsion or the like for injection. Alternatively, such a composition can be a dry product which can be made liquid with addition of a suitable carrier before use. The pharmaceutical compositions mentioned above can be prepared by usual methods.

In accordance with the form of the pharmaceutical composition obtained, the composition is administered via a suitable route. For example, those for injection are given intravenously, intramuscularly, subcutaneously, intracuteneously, intraperitoneally or otherwise. The solid composition is given orally or intraintestinally. The amount of the active component of the composition and the dosage of the composition are suitably determined according to the method and form of administration, purpose of use, symptoms of the patient, etc. and are not definitely determinable. It is generally desirable to incorporate about 1 to about 80 wt. % of the active component into the pharmaceutical preparation and to give the preparation at a daily dose of about 0.1 $\mu$g to about 10 mg, calculated as the active component, for adults. The preparation need not always be given only once a day but can be given in three to four divided doses daily.

The present invention will be described in greater detail with reference to the following reference examples wherein IL-1$\beta$ was prepared and examples wherein derivatives of the invention were prepared.

(1) Determination of IL-1 activity

Expressed in terms of LAF activity as measured by the method of J. J. Oppenhein et al. (J. Immunol., 116, 1466 (1976)) using thymus cells of a mouse of C3H/HeJ strain.

(2) Determination of GIF activity

Portions (0.1 ml) of the test solution diluted to varying concentrations were placed into the wells of 96-well microplate (Corning Co., Ltd.), 0.1 ml of Eagle's MEM suspension containing 10% FCS containing human melonoma cells A375 in an amount of $2 \times 10^4$ cells/ml was then placed into each well, and the cells were incubated in a $CO_2$ incubator (Napco Co., Ltd.) for 4 days. After the incubation, 0.05 ml of 0.05% Neutral Red (Wako Junyaku Co., Ltd.) was placed into each well, followed by incubation at 37°C for 2 hours. After removing the supernatant, 0.3 ml of phosphoric acid buffer saline was gently poured into each well for washing. After removing the washing, 0.1 ml of mixture of sodium phosphate monobasic and ethanol in equal amounts was placed into each well, the plate was shaken for several minutes by a micromixer, and the amount of pigment taken into the cell was measured at an absorbance of 540 m$\mu$

using a photometer for 96-well microtitration plates (Titer check multiscane, Flow Lab.) to determine growth inhibition activity. The test group exhibiting 50% of the inhibition of cell growth of the control group, i.e., the test group which exhibited 1/2 the absorbance measured of the control group, was identified. The reciprocal of the number of times of dilution for the test group was taken as the GIF activity unit. Accordingly, when the GIF activity is 10 units, for example, the test solution, if diluted tenfold, still has activity to inhibit cell growth 50%.

The following drawings are referred to in reference examples and examples.

Fig. 1 is a restriction enzyme map of plasmid pGIF-$\alpha$ cDNA;

Fig. 2 is a diagram showing how plasmid ptrpGIF-$\alpha$ is constructed from plasmid pGIF-$\alpha$ and plasmid pTMI;

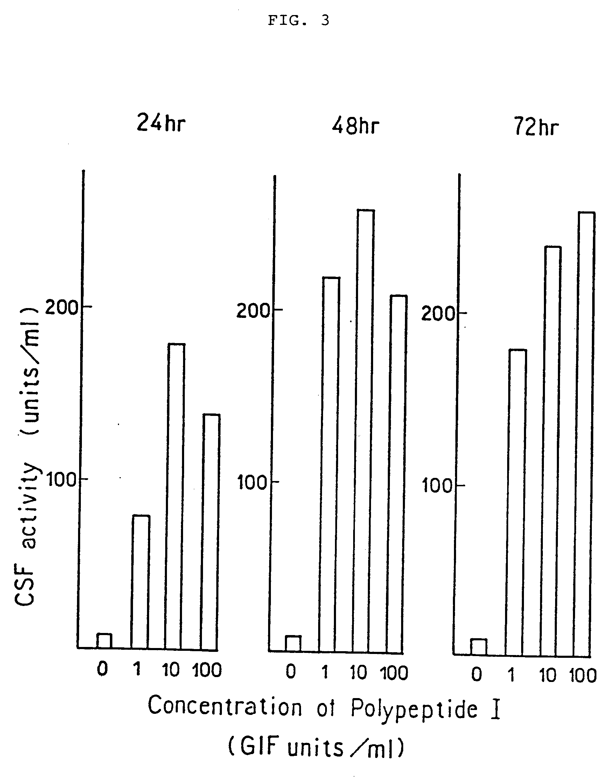

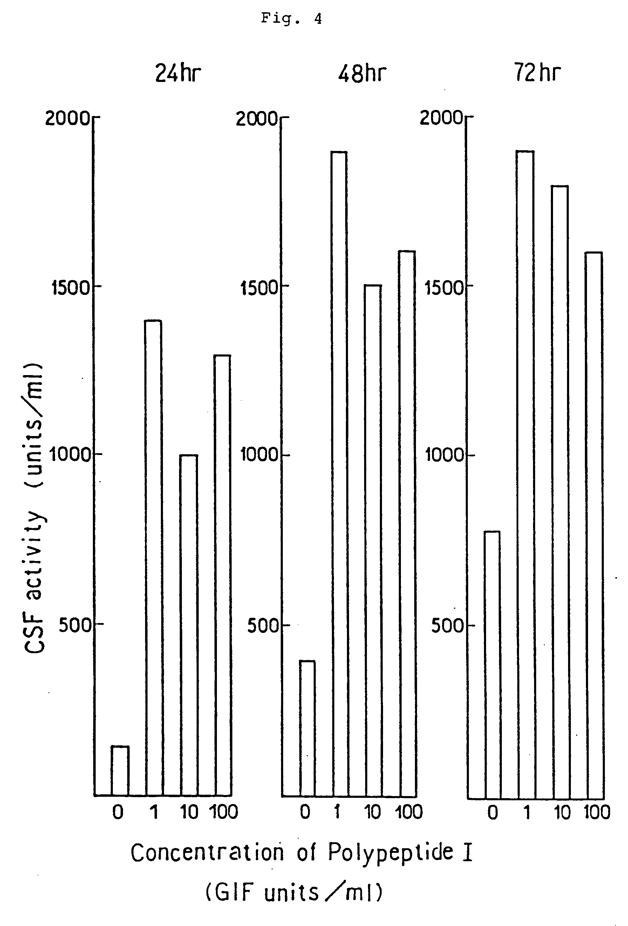

Figs. 3 to 5 are graphs showing the results obtained by testing polypeptide I (IL-1$\beta$) for its effect to promote production of CSF;

Fig. 6 is a graph showing the results obtained by testing polypeptide I (IL-1$\beta$) for anti-arthitis effect

## Reference Example 1

Preparation of plasmid pGIF-$\alpha$

### (1) Preparation of human lymphocytes

Human peripheral blood was collected, from which $1.9 \times 10^{10}$ lymphocytes were obtained by the Ficoll-Hypaque density gradient centrifugation method (Eur. J. Immunol. 4, 808 (1974)).

A quantity of the lymphocytes were suspended in RPMI 1640 medium containing 5% of human serum at a concentration of $4 \times 10^8$ cells/ml. The suspension was dividedly placed into Petri dishes, 9 cm in diameter, and the cells were incubated in the presence of 5% carbon dioxide gas at 37°C for one hour. The cells which were not adhered on the bottom of each dish were removed, and were stimulated with RPMI 1640 medium containing 10% FCS, 0.5 ng/ml of TPA (product of Sigma) and 10 $\mu$g/ml of LPS (product of Difco). After incubating the cells in 5% carbon dioxide gas at 37°C for 4 hours, $9 \times 10^8$ adhered lymphocytes were obtained using PBS and 0.02% EDTA.

### (2) Preparation of mRNA

Human adhered lymphocytes ($9 \times 10^8$ cells) obtained by the procedure (1) were dissolved in 30 ml of 6M guanidine thiocyanate solution (6 M guanidine isothiocyanate, 5 mM sodium citrate (pH 7.0), 0.1 M 2-mercarpto ethanol and 0.5% Sarkosyl), and the DNA was thereafter sheared with a 50-ml syringe barrel having G18G injection needle. A 12 g quantity of cesium chloride (CsCl) was completely dissolved in the solution. Portions (6.4 ml each) of the solution were superposed on 4 ml of 5.7 M CsCl (5.7 M CsCl-0.1M EDTA), and the combined layer was centrifuged by Beckman SW-40 Ti rotor at 31500 r.p.m. and 25°C for 20 hours. The RNA pellet sediment was washed with 70% ethanol and dissolved in TE solution (10 mM tris-HCl (pH 7.5), 1 mM EDTA). To the solution were added 3M sodium acetate (pH 5.2) and ethanol in amounts of 1/9 and 2.2 times the amount of the solution, respectively, and the mixture was allowed to stand at -70°C for one hour. The mixture was then centrifuged at 15000 r.p.m. and 4°C for 20 minutes to collect the RNA, which was then dissolved in TE solution.

In this way, 250 $\mu$g of total RNA was prepared from about $9 \times 10^8$ adhered lymphocytes.

To obtain mRNA from the RNA, the RNA was subjected to column chromatography using oligo(dT)-cellulose (Collaborative Research Inc.). For adsorption, a solution of 10 mM tris-HCl (pH 7.5), 0.5 M NaCl and 1 mM EDTA was used, and the column was washed with the same solution. The RNA was eluted with 10 mM tris-HCl (pH 7.5) and 1 mM EDTA.

Consequently 17.5 $\mu$g of mRNA was obtained.

### (3) Preparation of cDNA

From the mRNA obtained by the procedure (2), cDNA was synthesized in vitro, and recombinant DNA was prepared using Okayama-Berg plasmid vector (Okayama, H. and Berg, P., Mol. Cell. Biol., 2, 161 (1982)) and was transformed in E coli to obtain cDNA library. The procedures given below were followed.

(3-1) Preparation of vector primer and linker DNA

DNA (400 $\mu$g) of pBR322-SV40 (0.71-0.86) was digested with 700 units of KpnI (NEB) at 37°C for 5 hours. The reaction was terminated with a mixture of 40 $\mu$l of 0.25 M EDTA (pH 8.0) and 20 $\mu$l of 10% SDS. The reaction mixture was subjected to extraction with the same volume of phenol-chloroform (1:1). DNA was precipitated using ethanol, followed by centrifugation and washing with 70% ethanol to cellect DNA. The DNA obtained was dissolved in 200 $\mu$l of mixture of 140 mM sodium cacodylate, 30 mM tris-HCl (pH 6.8), 1 mM $CoCl_2$, 0.1 mM DTT and 0.25 mM dTTP (containing 0.5 $\mu$Ci of $\alpha$-$^{32}$P-dTTP). The solution was treated with 400 units of terminal transferase (TTase, PL) for 30 minutes to elongate the dT chain. The reaction was terminated with 20 $\mu$l of 0.25 M EDTA and 10 $\mu$l of 10 % SDS, followed by extraction with phenol-chloroform 4 times. DNA was collected by precipitation with ethanol. Consequently, the dT chain was lengthened by about 70 bases.

The DNA thus obtained was digested at 37°C for 6 hours with 17 units of HpaI (NEB) and electrophoresed with agarose (low-melting agarose, BRL, 1%) to collect about 2.7 kb DNA fragment.

After the electrophoresis, the DNA was stained with 0.5 $\mu$g/ml of ethidium bromide, the agarose containing the approximately 2.7 kb fragment was cut out under UV irradiation, and to the agarose was added 5 times the volume of 20 mM tris-HCl (pH 8.0)-1 mM EDTA to dissolve the agarose at 65°C over a period of 5 minutes, followed by extraction with phenol, then with phenol-chloroform (1:1) and thereafter with chloroform. The DNA was collected by precipitation with ethanol.

Subsequently, the vector primer DNA was purified by oligo(dA) cellulose column chromatography. The DNA was dissolved in 1 ml of 10 mM tris-HCl (pH 7.3)-1 mM EDTA-1 M NaCl buffer. The solution was ice-cooled, then placed on the column equilibrated with the same buffer, which was thereafter washed with 1 ml of the same buffer and subsequently returned to room temperature. Elution then followed with 10 mM tris-HCl (pH7.3)-1 mM EDTA to collect the peak fraction. The DNA was collected by precipitation with ethanol, then dissolved in 100 $\mu$l of 10 mM tris-HCl (pH 7.3)-1 mM EDTA and stored at 4°C.

Linker DNA was prepared by the following procedure. pBR322-SV40 (0.19-0.32) DNA (100 $\mu$g) was digested with 120 units of PstI (NEB) at 37°C for 1.5 hours. The termination of the reaction was followed by extraction with phenol-chloroform and precipitation with ethanol. The DNA collected was dissolved in 50 $\mu$l of mixture of 140 mM sodium cacodylate, 30 mM tris-HCl (pH 6.8), 1 mM $CoCl_2$, 0.1 mM DTT and 0.25 mM dGTP (containing 1 $\mu$Ci of $\alpha$-$^{32}$P-dGTP). The solution was acted on by 60 units of TTase for 20 minutes, whereby dG chain of 18 bases was attached. After the termination of the reaction, the DNA was collected, digested with 50 units of HindIII (Takara Shuzo Co., Ltd.) and electrophoresed with agarose (1.8%) in the same manner as above to collect about 0.28 kb DNA fragment and obtain 2.3 $\mu$g of linker DNA.

(3-2) Synthesis of cDNA and preparation of cDNA library

RNA (5 $\mu$g) was dried in a vacuo, then dissolved in 10 $\mu$l of 5 mM tris-HCl (pH 8.3) heated at 65°C for 5 minutes and immediately cooled to 37°C. To the reaction mixture was added 20 $\mu$l of mixture of 50 mM tris-HCl (pH 8.3), 8 mM $MgCl_2$, 30 mM KCl, 0.3 mM DTT, 2 mM dNTP and 10 $\mu$Ci of $\alpha$-$^{32}$P-dCTP. The resulting mixture was maintained at 37°C for 5 minutes.

Ten units of RTase (reverse transcriptase, product of Seikagaku Kogyo Co., Ltd.) was added to the mixture and reacted therewith at 37°C for 15 minutes. With addition of 10 units of RTase again, the mixture was maintained at the same temperature for 15 minutes. The reaction was terminated with 2 $\mu$l of 0.25 mM EDTA (pH 8.0) and 1 $\mu$l of 10% SDS, followed by extraction with phenol-chloroform. To the extract were added 20 $\mu$l of 4M ammonium acetate and 80 $\mu$l of ethanol, and the mixture was frozen at -70°C for 15 minutes, then thawed at room temperature and centrifuged at 15000 r.p.m. and 4°C for 10 minutes. The sediment was dissolved in 20 $\mu$l of 10 mM tris-HCl (pH 7.3). To the solution were added 19 $\mu$l of 4 M ammonium acetate and 80 $\mu$l of ethanol to reprecipitate.

The precipitate was collected, washed with 70% ethanol and dissolved in 15 $\mu$l of mixture of 140 mM sodium cacodylate, 30 mM tris-HCl (pH 6.8), 1 mM $CoCl_2$, 0.1 mM DTT, 0.2 $\mu$g of poly A and 66 $\mu$M of ($\alpha$-$^{32}$P)dCTP (10 $\mu$Ci). TTase (P.L., 18 units) was added to the solution and reacted therewith at 37°C for 5 minutes. The reaction mixture was rapidly cooled to 0°C, and the reaction was terminated with 1.3 $\mu$l of 0.25 M EDTA and 0.65 $\mu$l of 10% SDS, followed by extraction with phenol-chloroform and precipitation with ethanol.

The precipitate was collected by centrifugation and then digested with 4 units of HindIII (Takara Shuzo Co., Ltd.) at 37°C for 2 hours. Termination of the reaction was followed by extraction with phenol-chloroform and precipitation with ethanol. The precipitate was collected and dissolved in 10 $\mu$l of mixture of 10 mM tris-HCl (pH 7.3) and 1 mM EDTA. With addition of 3 $\mu$l of ethanol, the solution was preserved at -20°C.

One µl of the specimen thus obtained was maintained along with 5 ng of linker DNA in 10 µl of mixture of 10 mM tris-HCl (pH 7.5), 1 mM EDTA and 0.1 M NaCl at 65°C for 2 minutes and then at 42°C for 30 minutes, and thereafter cooled to 0°C. To the mixture was added 90 µl of a mixture solution of 20 mM tris-HCl (pH 7.5), 4 mM MgCl₂, 10 mM (NH₄)₂SO₄, 0.1 M KCl, 0.1 mM β-NAD, 50 µg/ml BSA-6 units/ml of E. coli DNA ligase, and then the combined solution was maintained at 12°C overnight.

To the solution were added 0.5 µl of 10 mM dNTP, 0.56 µl of 10 mM NAD, 0.5 µl of E. coli DNA polymerase I (product of Boehringer Mannheim) and 0.2 µl of RNase H (PL). The mixture was maintained at 12°C for one hour and then at 25°C for one hour, and thereafter frozen at -20°C for preservation.

E coli HB101 strain was incubated to $OD_{550}$ of 0.45 in LB medium (10 g of bacto-trypton, 5 g of bacto-yeast extract and 10 g/liter of NaCl). The culture was ice-cooled for 5 minutes and then centrifuged at 4°C at 8000 r.p.m. for 5 minutes to harvest the cells. The pellets of cells were suspended in an ice-cooled mixture of 30 mM potassium acetate, 100 mM RbCl, 10 mM CaCl₂, 50 mM MnCl and 15% glycerin, maintained at 0°C for 5 minutes and centrifuged at 4°C at 8000 r.p.m. for 5 minutes. The cells collected were suspended again in a mixture of 10 mM MOPS (morpholinopropanesulfonic acid), 75 mM CaCl₂, 10 mM RbCl and 15% glycerin and maintained at 0°C for 15 minutes to prepare competent cells, which were thereafter preserved at -70°C.

The frozen suspension was thawed at room temperature. The DNA specimen (20 µl) was added to a 400 µl portion of the suspension, and the mixture was allowed to stand at 0°C for 30 minutes, subjected to heat shock at 42°C for 90 seconds and then allowed to stand again at 0°C for 1 to 2 minutes. With addition of 2 ml of LB medium, the mixture was maintained at 37°C for 30 minutes. LB medium (50 times the volume of the mixture) was inoculated with the mixture, followed by incubation at 37°C for 6 hours. Ampicillin was added to the culture to a concentration of 50 µg/ml, followed by incubation again overnight, whereby cDNA library was prepared. The cDNA library was preserved in 50% glycerin at -20°C.

(3-3) Preparation of synthetic probe

```
        Ala - Pro - Val - Arg - Ser - Leu - Asn - Cys -

     5' GCC   CCC   GTG   AGG   TCC   CTG   AAC   TGC

     3' CGG   GGG   CAC   TCC   AGG   GAC   TTG   ACG

        Thr - Leu - Arg - Asp - Ser - Gln - Gln - Lys -

        ACC   CTG   AGG   GAC   TCC   CAG   CAG   AAG

        TGG   GAC   TCC   CTG   AGG   GTC   GTC   TTC

        Ser - Leu - Val - Met

        TCC   CTG   GTG   ATG - 3'

        AGG   GAC   CAC   TAC - 5'
```

A nucleotide sequence (shown in the lowest line) complementary to the base sequence of the above formula is synthesized by the following method to use the complementary sequence as a probe for selecting a transformant having cDNA coding for IL-1β. Thus, the fully protected DNA was synthesized by the solid-phase phosphite triester method wherein N,N-dialkylmethyl phosphoramidite derivative is used as a condensation unit (Nature, 310, 105 (1984)), using an automatic synthesizer (380A DNA Synthesizer, Applied Biosystems Inc., Foster City, California 94404, U.S.A.). Subsequently, the fully protected DNA was treated with 28% ammonia water at 55°C for 10 hours, whereby the protective groups (i.e., the acyl groups for the amino groups of A, G and C) other than DMTr (dimethoxytrityl) group attached to the hydroxyl group at the 5' terminal were removed to obtain partially protected DNA. Next, the partially protected DNA was purified by reverse-phase HPLC using $C_{18}$ column and then treated with 80% acetic acid at room temperature for 10 minutes to remove DMTr group. The nucleotide thus obtained was purified by electrophoresis using 10% polyacrylamide gel containing 7M urea and by Bio-gel P-30 (Bio-Rad Lab.) to obtain the desired DNA (60 mer).

The DNA (6 $\mu$g) thus obtained was reacted with 12 units of T4 polynucleotide kinase (Takara Shuzo) at 37°C for one hour in 50 $\mu$l of reaction mixture (50 mM tris-HCl (pH 7.6), 10 mM MgCl$_2$, 10 mM 2-mercapto ethanol, 0.2 mg/ml of fetal bovine thymus DNA and 50 $\mu$Ci ($\gamma^{32}$P)-ATP) to label the 5' terminal of the DNA. To separate the labeled DNA from the unreacted $^{32}$P, the reaction mixture was subjected to column chromatography using Biogel P-30 (Bio-Rad). The fraction of the labeled DNA was precipitated with 3M sodium acetate in 1/9 of the volume of the fraction and ethanol in 2.5 times the volume thereof. The precipitate was collected by centrifugation, dissolved in 400 $\mu$l of mixture of 10 mM tris-HCl (pH 8.0) and 1 mM EDTA and preserved at -20°C.

The specific activity of the resulting probe was at least $10^7$ cpm/$\mu$g DNA.

(3-4) Screening of cDNA library

Twenty-four nitrocellulose filters (Millipore HAIFO 8250), 80 mm in diameter, were placed over LB agar medium containing 50 $\mu$g/ml of ampicillin, and the cDNA library solution was spread over the filters, as so diluted that 5000 colonies were applied to each filter, followed by incubation overnight at 37°C.

A fresh nitrocellulose filter was placed over the filter where colonies appeared to prepare a replica filter.

The original filter (master filter) was preserved at 4°C. The replica filter was held on the same agar medium as above at 37°C for 6 hours for incubation and thereafter transferred onto LB agar medium containing 200 $\mu$g/ml of chloramphenicol, followed by incubation at 37°C overnight.

The filter was treated with 0.5 N NaOH, then with 1M tris-HCl (pH 8.0) and thereafter with a mixture of 1 M tris-HCl (pH 8.0) and 1.5 M NaCl. The filter was then dried in air and subsequently baked under a vacuum at 80°C for 2 hours.

The baked filter, while being lightly shaken, was maintained at 68°C overnight in 20 ml of solution of 1.2 M NaCl, 0.12 M trisodium citrate, 10 mg/ml of Ficoll, 10 mg/ml of polyvinylpyrrolidine, 10 mg/ml of BSA, 0.1% SDS and 1 mg/ml of salmon sperm DNA. The solution was replaced by a solution of 1.2 M NaCl, 0.12 M trisodium citrate, 10 mg/ml of Ficoll, 10 mg/ml of polyvinylpyrrolidine, 10 mg/ml of BSA, 0.1% SDS and $10^6$ cpm/ml of the probe, in which the filter was maintained at 42°C overnight with light shaking for hybridization.

After the hybridization, the filter was withdrawn from the solution, washed with a solution of 1.2 M NaCl, 0.12 M sodium citrate and 0.1% SDS three times at room temperature and thereafter washed with the same solution at 60°C until the count of the background of the filter became 200 cpm as determined by GM survey meter.

The filter was dried in air and then subjected to autoradiography at -70°C for 2 days using sensitized paper and x-ray film (Fuji RX).

After developing the film, the colonies present in the signal region were scraped off from the master filter, and the foregoing procedure was repeated to isolate the colonies with a positive signal.

Consequently, clone I-2 having a strong signal was isolated.

(3-5) Analysis of clone

Restriction enzyme map of plasmid pGIF-$\alpha$ cDNA contained in clone I-2 was prepared.

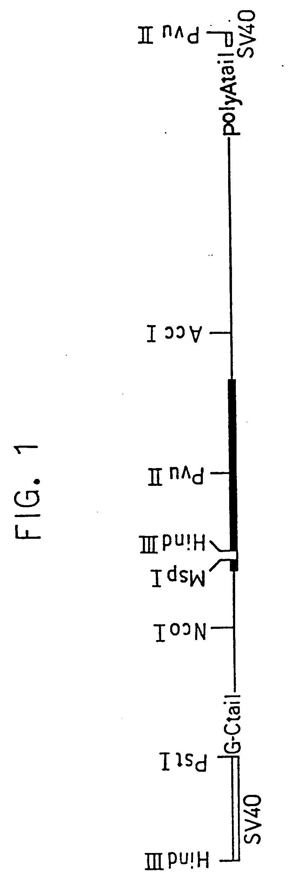

Fig. 1 shows the map.

Fig. 1 reveals that the cDNA has sites to be cleaved with NcoI (Nippon Gene), Hind III (Nippon Gene), PvuII (Nippon Gene) and AccI (Nippon Gene), one site by each, these cleavage sites being arranged in this order from the 5' terminus. It was found that the cDNA has a length of about 1.5 kb which is sufficient to code for IL-1$\beta$ having a molecular weight of about 18 kd.

Next, the base sequence of pGIF-$\alpha$ cDNA was determined by the Maxam-Gilbert chemical modification method (Meth. Emzym. 65, 499-566, 1980) and the dideoxynucleotide chain termination method using M13 phage (Messing, J. and Vieira, J., Gene, 19, 269-276 (1982)).

CTTATTACAGTGGCAATGAGGATGACTTG

TTCTTTGAAGCTGATGGCCCTAAACAGATGAAG
                 Met Lys

TGCTCCTTCCAGGACCTGGACCTCTGCCCTCTG
Cys Ser Phe Gln Asp Leu Asp Leu Cys Pro Leu

GATGGCGGCATCCAGCTACGAATCTCCGACCAC
Asp Gly Gly Ile Gln Leu Arg Ile Ser Asp His

CACTACAGCAAGGGCTTCAGGCAGGCCGCGTCA
His Tyr Ser Lys Gly Phe Arg Gln Ala Ala Ser

GTTGTTGTGGCCATGGACAAGCTGAGGAAGATG
Val Val Val Ala Met Asp Lys Leu Arg Lys Met

CTGGTTCCCTGCCCACAGACCTTCCAGGAGAAT
Leu Val Pro Cys Pro Gln Thr Phe Gln Glu Asn

GACCTGAGCACCTTCTTTCCCTTCATCTTTGAA
Asp Leu Ser Thr Phe Phe Pro Phe Ile Phe Glu

GAAGAACCTATCTTCTTCGACACATGGGATAAC
Glu Glu Pro Ile Phe Phe Asp Thr Trp Asp Asn

GAGGCTTATGTGCACGATGCACCTGTACGATCA
Glu Ala Tyr Val His Asp <u>Ala Pro Val Arg Ser</u>

CTGAACTGCACGCTCCGGGACTCACAGCAAAAA
<u>Leu Asn Cys Thr Leu Arg Asp Ser Gln Gln Lys</u>

AGCTTGGTGATGTCTGGTCCATATGAACTGAAA
<u>Ser Leu Val Met </u>Ser Gly Pro Tyr Glu Leu Lys

```
GCTCTCCACCTCCAGGGACAGGATATGGAGCAA
Ala  Leu  His  Leu  Gln  Gly  Gln  Asp  Met  Glu  Gln

CAAGTGGTGTTCTCCATGTCCTTTGTACAAGGA
Gln  Val  Val  Phe  Ser  Met  Ser  Phe  Val  Gln  Gly

GAAGAAAGTAATGACAAAATACCTGTGGCCTTG
Glu  Glu  Ser  Asn  Asp  Lys  Ile  Pro  Val  Ala  Leu

GGCCTCAAGGAAAAGAATCTGTACCTGTCCTGC
Gly  Leu  Lys  Glu  Lys  Asn  Lue  Tyr  Leu  Ser  Cys

GTGTTGAAAGATGATAAGCCCACTCTACAGCTG
Val  Leu  Lys  Asp  Asp  Lys  Pro  Thr  Leu  Gln  Leu

GAGAGTGTAGATCCCAAAAATTACCCAAAGAAG
Glu  Ser  Val  Asp  Pro  Lys  Asn  Tyr  Pro  Lys  Lys

AAGATGGAAAAGCGATTTGTCTTCAACAAGATA
Lys  Met  Glu  Lys  Arg  Phe  Val  Phe  Asn  Lys  Ile

GAAATCAATAACAAGCTGGAATTTGAGTCTGCC
Glu  Ile  Asn  Asn  Lys  Leu  Glu  Phe  Glu  Ser  Ala

CAGTTCCCCAACTGGTACATCAGCACCTCTCAA
Gln  Phe  Pro  Asn  Trp  Tyr  Ile  Ser  Thr  Ser  Gln

GCAGAAAACATGCCCGTCTTCCTGGGAGGGACC
Ala  Glu  Asn  Met  Pro  Val  Phe  Leu  Gly  Gly  Thr

AAAGGCGGCCAGGATATAACTGACTTCACCATG
Lys  Gly  Gly  Gln  Asp  Ile  Thr  Asp  Phe  Thr  Met
```

CAATTTGTGTCTTCCTAAAGAGAGCTGTACCCA
Gln Phe Val Ser Ser

GAGAGTCCTGTGCTGAATGTGGACTCAATCCCT

AGGGCTGGCAGAAAGGGAACAGAAGGTTTTTGA

GTACGGCTATAGCCTGGACTTTCCTGTTGTCTA

CACCAATGCCCAACTGCCTGCCTTAGGGTAGTG

CTAAGACGATCTCCTGTCCATCAGCCAGGACAG

TCAGCTCTCTCCTTTCAGGGCCAATCCCAGCCC

TTTTGTTGAGCCAGGCCTCTCTCTCACCTCTCC

TACTCACTTAAAGCCCGCCTGACAGAAACCAGG

CCACATTTTGGTTCTAAGAAACCCTCCTCTGTC

ATTCGCTCCCACATTCTGATGAGCAACCGCTTC

CCTATTTATTTATTTATTTGTTTGTTTGTTTTG

ATTCATTGGTCTAATTATTCAAAGGGGGCAAG

AAGTAGCAGTGTCTGTAAAAGAGCCTACTTTTT

ATTAGCTATGGAATCAATTCAATTTGGACTGGT

GTGCTCTCTTTAAATCAAGTCCTTTAATTAAGA

CTGAAAATATATAAGCTCAGATTATTTAAATGG

GAATATTTATAAATGAGCAAATATCATACTGTT

CAATGGTTCTCAAATAAACTTCACTAAAAAAAA

AAAAAAAAAAAAAAAAAAAAAAAA

The formula shows that the underlined region of the 312nd to the 371st nucleotides from the 5' terminal is complementary to the synthesized probe. The nucleotide sequence had 75% homology with the nucleotide sequence determined according to the human codon usage frequencies.

Further when the cDNA of pGIF-α was searched for the longest reading frame, this frame was found to be the region of the 57th to 771st nucleotides from the 5' terminal. This indicates that the cDNA of pGIF-α is cDNA coding for a human IL-1β precursor protein.

The transformant prepared by introducing the above plasmid, pGIF-α, into E. coli χ1776 has been deposited under the name of Escherichia coli χ1776/pGIF-α and the deposition number FERM BP-948 in Fermentation Research Institute, Agency of Industrial Science and Technology since December 12, 1985

Ref. Ex. 2 Preparation of Polypeptide I (IL-1β)

(1) The following oligodeoxynucleotides (I) and (II) were prepared by the procedures described below.

5' HOCGATAATGGCTCCTGTACGTTCTCTGAACTGCACTCTCOH 3'      (I)
5' HOCGGAGAGTGCAGTTCAGAGAACGTACAGGAGCCATTATOH 3'      (II)

5'-O-Dimethoxytrityl and N-protected deoxynucleoside (Applied Biosystems Inc.) attached to macroporous silica were used as starting materials. The nucleotide chain was successively lengthened from the 3' terminal toward the 5' terminal with the condensation units of 5'-0-dimethoxytrityl and N-protected deoxymononucleoside-3'-phosphoamidite, using an automatic synthesizer (380A DNA synthesizer, Applied Biosystems Inc.). The resulting product was treated with thiophenol for demethylation and further treated with 28% ammonia water at room temperature to remove the nucleotide from the silica, whereby fully protected oligonucleotide was obtained. The above procedure was executed entirely by the automatic synthesizer (Hunkapiller et al., Nature, 310, 105 (1984)).

The oligonucleotide was treated with 2 ml of 28% ammonia water at 55°C for 10 hours to remove the N-protective group and obtain 5'-O-dimethoxytrityloligonucleotide. One fifth of the amount of the product was purified by reverse-phase high-performance liquid chromatography using ODS (Yamamura Kagaku Kenkyusho Co., Ltd.) column and then treated with 150 μl of 80% acetic acid at room temperature for 20 minutes to obtain crude oligonucleotide. The product was further purified by reverse-phase high-performance liquid chromatography with ODS column to obtain the desired oligonucleotide.

The plasmid pGIF-α obtained by the procedure of Ref. Example 1, was cleaved with restriction enzymes AccI and ClaI to obtain a DNA fragment of about 1.2 kilo base pairs (kbp), which was isolated and purified by agarose gel electrophoresis. The DNA fragment was made blunt at the ends cleaved with restriction enzymes AccI and ClaI, using DNA polymerase I (Klenow fragment).

On the other hand, BamHI linker (5' HOCGGATCCGOH 3') was phosphorylated at the 5' terminal with T4 polynucleotide kinase and joined to the blunt-ended DNA fragment with T4 DNA ligase, followed by digestion with restriction enzyme BamHI and further with restriction enzyme MspI. The resulting reaction product was electrophoresed on agarose gel to isolate purified MspI-BamHI DNA fragment of about 540 bp.

The oligodeoxynucleotides (I) and (II) synthesized above were phosphorylated at the 5' terminal with T4 polynucleotide kinase and joined to MspI-BamHI DNA fragment using T4 DNA ligase, followed by digestion with restriction enzymes BamHI and ClaI. The reaction product was electrophoresed on agarose gel to isolate purified ClaI-BamHI DNA fragment of about 580 bp.

On the other hand, plasmids pTMI (Fumio Imamoto, Taishya, Vol. 22, 289 (1985)) were cleaved with restriction enzymes BamHI and ClaI, followed by agarose gel electrophoresis to isolate and purify DNA fragment of about 4.4 kbp having a trp promotor region. The DNA fragment and the ClaI-BamHI DNA fragment of about 580 bp prepared above were ligated with T4 DNA ligase to obtain the desired plasmid ptrpGIF-α for express polypeptide I.

The plasmid was introduced into E. coli HB101 for transformation, and the desired transformant E. coli HB101/ptrpGIF-α was selected by the restriction enzyme analysis of the plasmid DNAs which was obtained by the boiling method (T. Maniatis, E.F. Fritsch and J. Sambrook, Molecular Cloning, p. 366, Cold Spring Harfor Laboratory, (1982)).

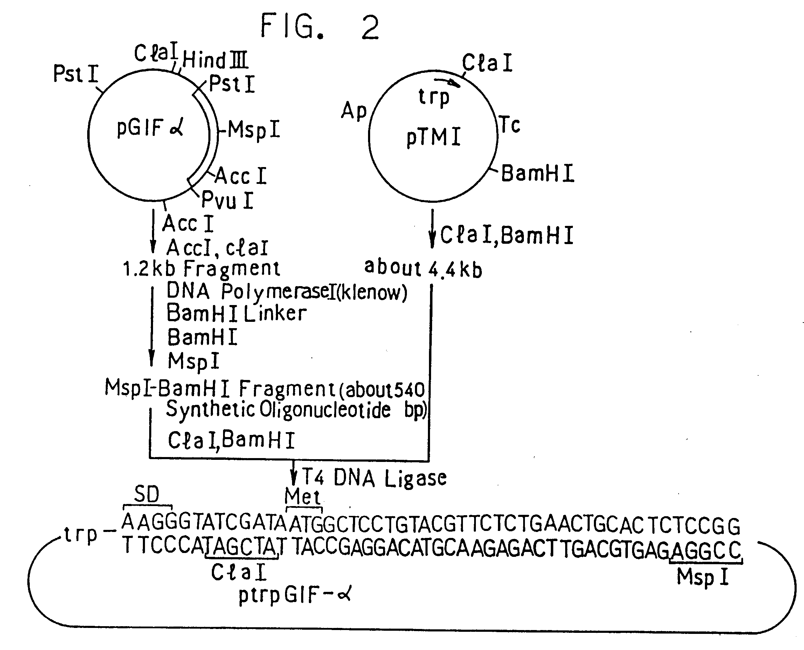

Fig. 2 schematically shows the above procedure.

The transformant prepared by introducing the plasmid ptrpGIF-α into E. coli χ1776 has been deposited under the name of Escherichia coli χ1776/ptrpGIF-α and deposition number FERM BP-949 in Fermentation Research Institute, Agency of Industrial Science and Technology since December 12, 1985.

(2) Culture of transformant

The transformant obtained above, i.e., E. coli HB101/ptrpGIF-α was incubated overnight at 37°C with shaking in 10 ml of LB medium (1% tryptone, 0.5% yeast extract and 0.5% NaCl) containing 50 $\mu$g/ml of ampicillin and 20 $\mu$g/ml of L-tryptophan. One-ml portion of the culture was inoculated in 50 ml of M9 minumum medium (0.6% $Na_2HPO_4$, 0.3% $KH_2PO_4$, 0.05% NaCl, 0.1% $NH_4Cl$, 2 mM $MgSO_4$, 0.2% glucose and 0.1 mM $CaCl_2$) containing 50 $\mu$g/ml of ampicillin and 1% Casamino acid and incubated at 37°C with shaking. The cells were harvested when the absorbance (O.D.) at 550 nm reached 1.0 and suspended in 5 ml of a solution of 15% sucrose, 50 mM tris-HCl (pH 8.0) and 50 mM EDTA (pH 8.0). A 500 $\mu$l of 10 mg/ml of lysozyme (as dissolved in 10 mM tris-HCl (pH 8.0)) was added to the suspension, and 5 ml of a solution of 0.3% Triton X100, 187.5 mM EDTA (pH 8.0) and 150 mM tris-HCl (pH 8.0) was further added. The mixture was allowed to stand at room temperature for 15 minutes, then thoroughly stirred and centrifuged to obtain a GIF activity positive-supernatant of cell extract.

(3) Purification of Polypeptide I

Ion-exchange chromatograph (CM-HPLC)

The cell extract supernatant prepared as above was dialyzed with 50 mM sodium acetate buffer (pH 5.5), and the dialyzate was subjected to ion-exchange chromatography (CM-HPLC) using Gilson high permeation liquid chromatography system (Gilson) under the following conditions.

Column :               IEX-535CM (6.0 x 150 mm, Toyo Soda Co., Ltd.)
Eluent A:              50 mM sodium acetate (pH 5.5)
Eluent B:              50 mM sodium acetate (pH 5.5) containing 0.5 M NaCl
Flow rate             : 0.5 ml/min.
Fraction volume:       Retention time

| 0-60 min | 2 ml/4 min/tube |
|---|---|
| 60-120 min | 0.5 ml/min/tube |
| 120-180 min | 2 ml/4 min/tube |

Concentration gradient:

| Time (min) | % B |
|---|---|
| 0 | 0 |
| 40 | 0 |
| 120 | 20 |
| 140 | 100 |
| 165 | 100 |
| 170 | 0 |

Reverse-phase high-performance liquid chromatography

The CM-HPLC procedure resulted in a GIF active fraction with a retention time of 90 to 91 minutes.

The active fraction was then subjected to reverse-phase high-performance liquid chromatography under the following conditions.

15

Column : C$_4$ Highpore reverse-phase column (RP304, Bio-Rad, 250 mm x 4.6 mm (diam.)

Eluents : A liquid = 0.1% TFA
B liquid = acetonitrile-1% TFA (9:1)

Flow rate : 1 ml/min.

Chart speed : Retention time

| 0-50 min | 5 min/cm |
|---|---|
| 50-80 min | 2 min/cm |

Concentration gradient:

| Time (min) | % B |
|---|---|
| 0 | 0 |
| 5 | 0 |
| 15 | 20 |
| 75 | 45 |
| 80 | 100 |
| 85 | 100 |
| 90 | 0 |

Fraction volume: 2 ml/2 min/tube

There was obtained at a retention time of 63.9 to 65.3 minutes the desired Polypeptide I exhibiting a single protein absorbance peak matching that of GIF activity.

The Polypeptide I thus obtained possess IL-1 activity and the specific activity was 2.7 x 10$^7$ GIF units/mg protein.

(4) Identification of Polypeptide I

SDS polyacrylamide gel electrophoresis (SDS-PAGE)

The SDS-PAGE analysis of Polypeptide I obtained by the above procedure (3) was conducted in accordance with the method of Laemmli, U.K. (Nature, 277, 680 (1970)) under the following conditions.

Specimen: The Polypeptide I fraction obtained by the reverse phase HPLC procedure was completely dried, then dissolved in Laemmli sample buffer (not containing 2-mercapto ethanol (2ME$^-$) or containing 2-mercapto ethanol in an amount of 1/20 the volume of the buffer (2ME$^+$)) and treated at 100°C for 4 minutes.

Gel : 15% Polyacrylamide gel 1.5 mm in thickness

Apparatus : Protean, product of Bio-Rad

Electrophoresis : 40 mA constant current for 2 hours

The gel resulting from the electrophoresis was stained with Silver Stain Kit (Bio-Rad).

The results indicate that Polypeptide I is migrated as a single band at the position of about 17Kd in the case of 2ME$^+$ or of about 17.5Kd in the case of 2ME$^-$.

Isoelectrofocussing (IEF)

Polypeptide I was subjected to IEF using PAG plate(LKB) 3.5 to 9.5 in pH range, and Model 1415 (Bio-Rad) under the following conditions.

Specimen: Four lanes were used, i.e. 0.037 $\mu$g, 0.074 $\mu$g and 0.74 $\mu$g portions of polypeptide I obtained by the foregoing procedure (3) and the following marker proteins (pI marker protein).

Marker proteins

Amyloglucosidase (3.50)
Soybean trypsin inhibitor (4.55)
$\beta$-lactoglobulin A (5.20)
Bovine carbonic anhydrase B (5.85)

16

Human carbonic anhydrase B (6.55)
Horse myoglobin-acidic band (6.85)
Horse myoglobin-basic band (7.35)
Lentil lectin-acidic band (8.15)
Lentil lectin-middle band (8.45)
Lentil lectin-basic band (8.65)
Tryspinogen (9.30)

Electrode solutions: Anode solution = 1M $H_3PO_4$
Cathode solution = 1M NaOH

Electrophoresis : With constant power of 1W/cm gel width with cooling (10°C) for 90 minutes
Staining: With silver stain Kit

The gel resulting from the electrophoresis was sliced at a spacing of 1 cm, subjected to extraction with use of 1 ml of distilled water with shaking (for two days) and then checked for pH to calculate the isoelectric point.

Polypeptide I had an isoelectric point (PI) of 6.8 ± 0.1 and appeared as a single band at this position.


Amino acid composition


The Polypeptide I fraction (30 μl) obtained by the reverse-phase high-performance liquid chromatographic procedure (3) was carefully placed into the bottom of a thick-walled hard test tube made of Pyrex glass and 12 mm x 120 mm, and was dried in a vacuum in a desiccator containing sodium hydroxide pellets. A 50 μl quantity of 4N methanesulfonic acid (containing 0.2% 3-(2-aminoethyl)indole and produced by Pierce) was added to the dry specimen within the tube. The interior of the tube was deaerated at 0.1 to 0.2 mm Hg for 1 minute and then sealed off. The specimen was hydrolyzed in a heater at 118°C over a period of 24 hours. After opening the tube, the mixture was neutralized with 46 μl of 4N sodium hydroxide and diluted to an amount of 450 μl with citric acid buffer.

A 250 μl quantity of the specimen solution was used for amino acid analysis by an amino acid analyzer (Model HITACHI 835, product of Hitachi Ltd.). The amino acids separated were detected by the o-phthalaldehyde method and quantitatively determined with reference to calibration curves prepared with use of authentic amino acids.

Table 1 shows the results in terms of the mole ratio of component amino acids based on Phe (9 moles). Under the above analysis conditions, Pro and Cys are not determinable. For Ser, Thr and Met, the percent recovery as achieved under the above conditions is given in parentheses.


Table 1

| Amino acid | Mole ratio |
|---|---|
| Asp and/or Asn | 17.1 |
| Ser | 10.9 (80%) |
| Thr | 5.4 (90%) |
| Glu and/or Gln | 23.8 |
| Gly | 8.3 |
| Ala | 5.0 |
| Val | 10.8 |
| Met | 5.5 (90%) |
| Ile | 4.9 |
| Leu | 15.1 |
| Tyr | 3.9 |
| Phe | (9) |
| Lys | 14.9 |
| His | 0.9 |
| Trp | 0.8 |
| Arg | 3.0 |

Amino acid sequence

A 150 $\mu$l quantity of Polypeptide I fraction obtained by the reverse-phase high-performance chromatographic procedure (3) was analyzed by a protein sequencer (Applied Biosystems Inc.). Each resulting PTH-amino acid was suitably diluted with 100 to 50 $\mu$l of 33% aqueous acetonitrile solution, and 5-$\mu$l portion of the dilution was injected into a chromatographic column by an autosampler,Waters 710B. For the chromatographic system, two pumps, Beckman Model 112, were operated by a controller, Model 421. The column used, measuring 2 mm x 250 mm and packed with Ultrasphere ODS-5 $\mu$m, was maintained at 55°C by a column heater. The flow rate was 0.3 ml/min. A mixture of 20 mM sodium acetate and acetonitrile was used for gradient elution. Absorbance was monitored at 269 nm. Analysis was conducted for 45 minutes.

The results of 20 cycles of analysis revealed that Polypeptide I obtaned by the procedure (3) had the following sequence of 20 amino acids at the N terminal.

```
Ala-Pro-Val-Arg-Ser-Leu-Asn-Cys-Thr-Leu-
Arg-Asp-Ser-Gln-Gln-Lys-Ser-Leu-Val-Met-
```

Ser was confirmed by one of by-products and was further confirmed as a dehydro form showing absorption at 322 nm. In cycle 8, the peak of by-product appeared to indicate Cys, and Cys was further identified by analyzing the original specimen after carboxamidemethylation.

Further polypeptide I was digested with trypsin to obtain peptide fragments. Amino acid composition analysis of the fragments revealed that the C terminal peptide was accurately contained in the polypeptide free of damage.

Stated more sepcifically, a 60 $\mu$g portion of polypeptide I was dissolved in 600 $\mu$l of 1% ammonium hydrogen carbonate. To the solution was added 20 $\mu$l of a solution of trypsin (0.2 mg/ml, product of Cooper Biomedical) in 1% ammonium hydrogen carbonate. The mixture was allowed to stand at 37°C for 24 hours to obtain peptides cleaved with tripsin. The peptide mixture was subjected to reverse-phase HPLC (C-18, 300Å, 4.6 x 150 mm; with use of 0.1% TFA as A solution and acetonitrile containing 1% TFA in 1/10 of the volume of the nitrile as B solution while increasing the amount of B solution at a rate of 1%/3 min for elution). All the components were isolated by the time the proportion of B solution increased to 40%. The peptides were subjected to amino acid analysis, whereby all the anticipated peptide fragments were identified. Especially, the C terminal peptide was found free from basic amino acids, and was found to contain the anticipated amino acids accurately by the analysis. Consequently, polypeptide I obtained by the procedure (3) had an accurate C terminal as anticipated.

In the same manner as above, the peptide fragments were checked for amino acid sequence. All the sequences confirmed were found to match the sequence of IL-1$\beta$. The confirmed sequences of the peptide fragments checked are shown below in amino acid numbers of IL-1$\beta$.

1-4, 5-11, 12-16, 17-27, 28-63, 64-65, 66-74, 75-88, 89-92, 95-98, 99-103, 104-109, 110-138, 139-153.

These results revealed that polypeptide I obtained by the procedure (3) was polypeptide I having the specified sequence. The calculated molecular weight of polypeptide I is 17376.59.

Example 2

(1) The transformant (E. coli HB101/ptrpGIF-$\alpha$) obtained in Reference Example 2-(1) was incubated overnight at 37°C in 400 ml of LB medium containing 50 $\mu$g/mg of ampicillin and 20 $\mu$g/ml of L-tryptophan. M9 minimum medium (20 liters) containing 1% Casamino acid was inoculated with the culture (400 ml), followed by incubation at 37°C for 8.5 hours.

The resulting culture was centrifuged to collect the cells, which were suspended in 1M $Na_2HPO_4$ and allowed to stand overhight in a cool chamber. The suspension was thereafter dialyzed against 10 mM tris-HCl buffer (pH 8.0) for 2 days.

The dialyzate obtained was centrifuged (10000 r.p.m., 30 minutes) to obtain a supernatant.

The supernatant was collected in an amount correspodning to 300 liters of E. coli culture, adjusted to a pH of 5.0 with 100 mM acetic acid and passed through Zeta Prep SP-250 cartridge (product of LKB) at a flow rate of 30 ml/min. The elution was conducted with 10 mM sodium acetate (pH 5.3) for 100 minutes, then with 50 mM sodium acetate (pH 5.5) containing 0.1 M NaCl for 220 minutes, and further with 50 mM sodium acetate (pH 5.5) containing 1M NaCl. The fractions were checked by HPLC to collect the fractions

18

containing the desired substance, i.e. fractions No.8 to No.10 of 50 mM sodium acetate (pH 5.5) containing 0.1 M NaCl.

The combined fraction was adjusted to pH 4.5 with 100 mM acetic acid, subjected to HPLC (TSK Gel SP-5PW, 5.5 x 20 cm, product of Toyo Soda Mfg. Co., Ltd.) and to elution under the following conditions to obtain fractions of 65 to 72 minutes in retention time (r.t.).

Eluent A: 50 mM sodium acetate (pH 5.5)
Eluent B: 50 mM sodium acetate (pH 5.5) containing 0.5 M NaCl
Flow rate: 30 ml/min
Concentration gradient:

| Time (min) | % B |
|---|---|
| 0 | 0 |
| 30 | 0 |
| 84 | 9 |
| 104 | 100 |
| 124 | 100 |
| 134 | 0 |
| 180 | 0 |

Thus, polypeptide I was obtained as purified.

By ultrafiltration (YM-5 membrane), the product was concentrated to 20 mg/ml in the form of a solution of 20 mM sodium phosphate buffer (pH 7.0) by changing the buffer.

The concentrate was further passed through a filter apparatus equipped with Posidyne filter NFZ (product of Nohon Pall) for sterilization to obtain a pure product which was up to 100 pg/mg protein in pyrogen content.

The purification process afforded about 3 g of polypeptide I from 300 liters of E. coli culture.

Table 2

| Mole rato | |
|---|---|
| Amino acid | Polypeptide I |
| Asp and/or Asn | 17.7 |
| Ser | 13.0 |
| Thr | 5.8 |
| Glu and/or Gln | 24.0 |
| Gly | 8.6 |
| Ala | 5.2 |
| Val | 10.3 |
| Met | 5.8 |
| Ile | 4.9 |
| Leu | 15.5 |
| Tyr | 3.6 |
| Phe | (9) |
| Lys | 14.9 |
| His | 1.0 |
| Trp | present |
| Arg | 2.9 |

(4) Polypeptide I has two Cys residues (at the 8- and 71-positions) within the molecule. The state of SH group present on the side chain of these Cys residues was checked by the following method.

a. About 5 nmoles of polypeptide I obtained in Reference Example 2-(3) (in the form of 100 $\mu$l of solution of 50 mM sodium acetate (pH 5.5) containing 0.1 M NaCl) was placed into each of three test tubes A, B and C. A 300 $\mu$l quantity of 6 M guanidine hydrochloride solution containing 0.1 M tris-HCl (pH 8.45) was further placed into each tube.

19

Next, 25 $\mu$l of 6 M guanidine hydrochloride solution was placed into each of the tubes A and B to prepare blanks. Into the test tube C was placed 25 $\mu$l of 6 M guanidine hydrochloride solution containing 2 $\mu$moles of dithiothreitol serving as a reducing agent. Nitrogen gas was introduced into each test tube for 1 minute, and the tube was thereafter allowed to stand at 50°C for 2 hours.

Subsequently, 25 $\mu$l of 6 M guanidine hydrochloride solution was placed into the blank test tube A, and 25 $\mu$moles of 6 M guanidine chloride solution containing 4 $\mu$moles of iodoacetamide into each of the test tubes B and C. After introducing nitrogen gas into each test tube for 1 minute, the tube was allowed to stand in the dark at 25°C for 30 minutes.

With addition of 5 $\mu$l of 10% TFA to the mixture in each of the test tubes A, B and C thus prepared, the mixture was subjected to reverse-phase HPLC ($C_3$) to purify the protein.

About 1/10 of the quantity of purified protein was placed into a test tube, dried, hydrolyzed with 4N methanesulfonic acid and checked for amino acid composition.

Consequently, nearly equal quantities of carboxymethylcysteine were detected from the test tubes B and C, whereas polypeptide I in the test tube A remained unchanged. This indicates that the polypeptide in the test tubes B and C was converted to S-carboxamidemethylated polypeptide I (since the polypeptide in the test tube B was not exposed to the reducing agent), therefore revealing that no disulfide (S-S) linkage was formed by the Cys residues in polypeptide I.

Further the product remaining in each of the test tubes A, B and C was dried and then dissolved in 600$\mu$ I of 1% ammonium bicarbonate solution. The solution was adjusted to a concentration of 1 $\mu$g/5 $\mu$l. A trypsin solution (7.5 $\mu$l) was added to the solution to digest the protein with the enzyme at 37°C for 20 hours.

The enzymatic reaction was terminated by addition of 10 $\mu$l of 10% TFA to the mixture, and peptide fragments were separated off by reverse-phase HPLC ($C_{18}$).

As a result, the test tubes B and C exhibited nearly the same pattern, while the test tube A showed a pattern different therefrom.

The peptides thus isolated were checked for amino acid composition in the same manner as above. The peptide fragment in the test tube A revealed the presence of peptide which would not occur in the absence of Cys.

These results demonstrated that no disulfide linkage was formed intramolecularly or intermolecularly by the Cys residue of polypeptide.

b. The SH groups in polypeptide I were quantitatively determined by the Ellman method (Arch. Biochem. Biophys., 82, 70 (1959)) in the following manner.

A 200 $\mu$g quantity (11.5 $\mu$moles) of polypeptide I obtained in Reference Example 2-(3) was dissolved in 1 ml of 0.1 M tris-HCl buffer (pH 8.0) containing 6 M guanidine hydrochloride and 1 mM EDTA. On the other hand, fresh 0.05 M phosphate buffer (pH 7.0) was prepared which contained 0.01 M DTNB (5,5'-dithiobis(2-nitrobenzoic acid). (The buffer will hereinafter be referred to as the "Ellman buffer.")

One ml of 0.1 M tris-HCl buffer (pH 8.0) containing 6N guanidine hydrochloride and 10 mM EDTA was placed into a control cell, and 1 ml of the above solution containing polypeptide I into a specimen cell. A 40 $\mu$l quantity of the Ellman reagent was admixed with the contents of each cell, whereupon the absorbance of the mixture was measured at 412 nm. After a maximum absorbance was achieved, the reduction in the absorbance due to the decomposition of DTNB was corrected to "0" in determining the concentration of SH groups.

The absorbance actually obtained at 412 nm was 0.328.

On the other hand, $\epsilon_{412}$ of 3-carboxylate-4-nitrothiophenolate ion in an aqueous solution containing 6 M guanidine hydrochloride was 13880 M$^{-1}\cdot$cm$^{-1}$ (Eur. J. Biochem., 30, 32 (1972)). This revealed that the SH groups in the polypeptide I solution were 0.0000245 in M/e and that 11.5 $\mu$moles of polypeptide I contained 24.5 $\mu$moles of SH groups.

The above result demonstrated that the Cys residues contained in polypeptide I had a free SH group.

Further little or no change was found in GIF activity and in the quantity of SH groups determined by the Ellman method when a solution of polypeptide I in water or in PBS (-) was repeatedly lyophilized 3 to 4 times.

(5) When a hetero protein is expressed in a large amount in E. coli or the like by a gene recombination technique, the protein is not infrequently accumulated as an inclusion body in E. coli cells. In such a case, collection of the protein from the cells requires a treatment under severe conditions which, for example, involve use of a denaturing agent such as 7 M guanidine hydrochloride, 8 M urea, 0.1% SDS, or the like. Such a treatment, however, is very likely to irreversibly cause damage to the desired protein including a higher structure thereof. Accordingly, it is a matter of great interest in gene recombination techniques to isolate the desired protein under mild conditions without using the denaturing agent to the greatest possible extent. In this respect, the method (1) described above is very favorable since the desired protein can be extracted and isolated under a very mild condition, i.e. by osmotic shock. The method is desirable also because the desired protein obtained has a higher structure resembling that of natural product.

While the GIF activity of IL-1$\beta$ (polypeptide I) of the invention has been described, the following tests were conducted.

Table 5 shows the LAF activity (U) per mg of polypeptides, calculated as protein of polypeptide I, as determined by the RIF method using the aforementioned antiserum against polypeptide I.

Table 5

| Polypeptide | LAF activity (U/mg) |
|---|---|
| Polypeptide I | $4.5 \times 10^6$ |

Pharmacological Test Example 1

Testing polypeptide I for effect to induce CSF production

(1) Test of inducing effect on CSF production by human lung cells

The following test was conducted using human embryonic lung fibroblasts (HFL-1, ATCC registered cell strain No.CCL-153) which produce CSF.

HFL-1 cells were suspended in Ham's F-12K culture (Ham, R.G., Proc. Natl. Acad. Sci., 53, 288 (1965) containing 10% FCS to a concentration of $2 \times 10^5$ cells/ml.

Polypeptide I obtained in the foregoing reference example was added to portions of the cell suspension in varying concentrations. Each of the mixtures was incubated in a carbon dioxide gas incubator at 37 °C for 24, 48 or 72 hours. The culture supernatant was collected, and the amount of CSF produced and accumulated in the supernatant was measured using mouse bone marrow cells (Lewis, I.C. et al, J. Immunol., 128, 168 (1982)).

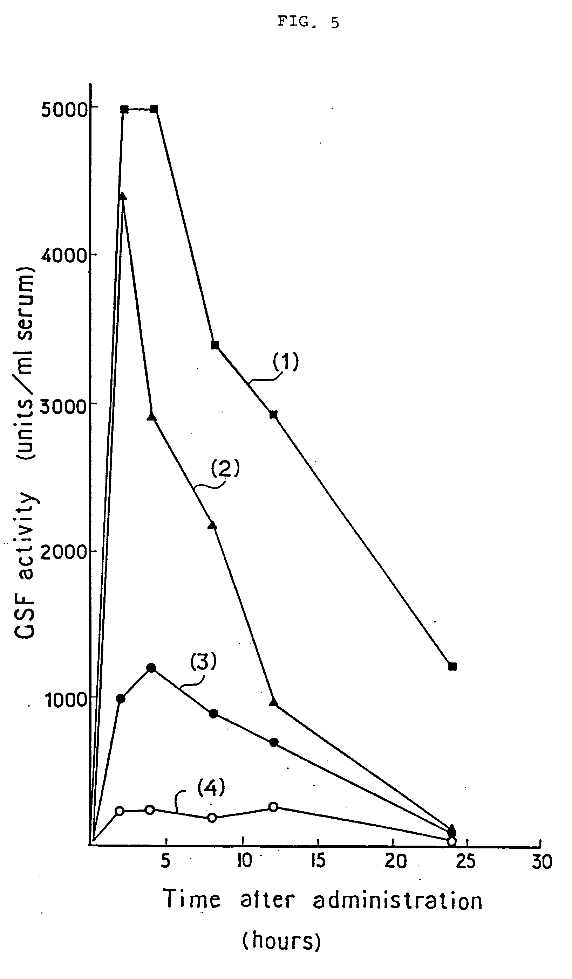

Fig. 5 shows the results achieved by polypeptide I during different periods of incubation. In the diagram, the concentration (GIF units/ml) of polypeptide I is plotted as abscissa vs. the CSF activity (units/ml) as ordinate.

The results achieved reveal that the addition of polypeptide I increases the amount of CSF produced by HFL-1 cell strain to as many as hundreds of times the amount produced in the absence of the polypeptide.

(2) Test of inducing effect of polypeptide I on CSF production by cells derived from human skin

The following test was conducted using normal human skin-derived cell strain, CRT-1445 (ATCC No.)

Cells of the above strain were suspended in Dulbeco MEM culture medium (Dulbeco, R. and Freeman, G., Virology, 8, 396 (1959)) containing 10% FCS to a concentration of $2 \times 10^5$ cells/ml.

Polypeptide I obtained in the foregoing reference example was added to portions of the cell suspension in varying concentrations. Each of the mixtures was incubated in a carbon dioxide gas incubator at 37 °C for 24, 48 or 72 hours. The culture supernatant was collected, and the amount of CSF produced and accumulated in the supernatant was measured using mouse bone marrow cells as in the test (1).

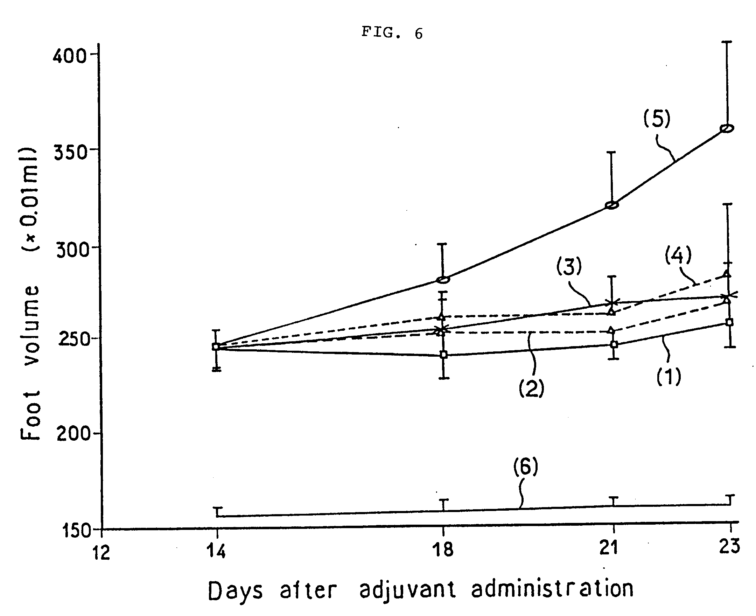

Fig. 6, like Fig. 5, shows the results achieved.

Fig. 6 shows that the addition of polypeptide in an amount of at least one unit/ml calculated as GIF activity to fibroblasts derived from the normal human skin gives the cells remarkably enhanced ability to produce CSF.

(3) Test of inducing effect on CSF production in vivo

The following animal experiment was conducted to substantiate that polypeptide I, when administered to the animal, acts to induce the production of CSF in vivo.

To normal mice (BALB/C strain, purchased from Experimental Animal Cooperative Association of Shizuoka Prefecture, Japan) was intravenously given polypeptide I obtained in Reference Example 2 in varying amounts ($10^3$ to $10^5$ units/animal calculated as GIF activity). Blood was collected from the animal 2,

EP 0 569 042 A1

4, 8, 12 and 24 hours after the administration and checked for the concentration of CSF in the serum using mouse bone marrow cells.

The results are given in Fig. 7, in which the time (hours) elapsed after the administration is plotted as abscissa vs. the CSF activity (units/ml serum) as ordinate. In the diagram, (1) represents a group to which polypeptide I was given at a dose of 100,000 GIF units/animal, (2) represents a group with a dose of 10,000 GIF units/animal, (3) represents a group with a dose of 1,000 GIF units/animal, and (4) represents a control group to which HSA was given at a dose of 10 µg/animal.

Fig. 7 reveals that polypeptide I, when given to animals, remarkably increases the CSF concentration of the serum of the animal. It is seen that polypeptide I greatly promotes the production of CSF in vivo in proportion to the dose.

Pharmacological Test Example 2

Rats with adjuvant arthritis were prepared by the method of Pearson (Pearson, C. M., Proc. Soc. Exp. Biol. Med., 91, 95 (1956)), Ward and Jones (Ward, J. R. and Jones, R. S., Arthritis Rheumatism, 5, 557 (1962)). More specifically, 0.05 ml of an adjuvant prepared by suspending dead cells of Mycobacterium butyricum in fluid paraffin was intracuteneously injected into the base portion of the tail of each of male rats of S.D. strain. On the 14th day, the animals were grouped (n = 6) according to the degree of swelling of the foot. During the period of the following 5 days, polypeptide I obtained in the reference example or the solvent therefor (saline for the control group) was intracutaneously given to the animals. The volume of the foot was measured from day to day to evaluate the influence of the polypeptide on arthritis.

The results are shown in Fig. 8, in which the number of days after the administration of the adjuvant is plotted as abscissa vs. the foot volume (x 0.01 ml) as ordinate. In the diagram, a group to which polypeptide I was given at a dose of 100,000 GIF units/animal is represented by (1), a group with a dose of 10,000 GIF units/animal by (2), a group with a dose of 1,000 GIF units/animal by (3), a group with a dose of 100 GIF units/animal by (4), the control group with saline by (5), and a group of normal rats by (6).

Fig. 8 shows that the foot swelling became aggravated until the 23rd days in the control group (5) but that polypeptide I was found to exhibit swelling inhibiting activity on the groups (1) to (4) to which the polypeptide was given, on the 4th days (after the administration, i.e. 18th day after the administration of the adjuvant) and during the following period. Even 4 days after the administration of the final dose (i.e. on the 23rd day after the administration of the adjuvant), the polypeptide was found still effective for preventing progress of arthritis.

When cytokines are to be produced from animal cells, it is essential that the polylpeptide of the invention used for inducing the production be stable in structure under the prevailing condition and be bound to the IL-1 receptor on the surface of the cells. More specifically, it is required that the present polypeptide bind to the IL-1 receptor and transmit to the cell a singal necessary for the production of the cytokine.

Accordingly, the following test was conducted in connection with the binding of the present polypeptide to the IL-1 receptor on fibroblasts.

Balb/3T3 cells (clone A31: ATCC, CCL-163, 1 x 10$^6$ cells/well) almost uniformly grown over a 6-well plate were reacted at 4°C for 2 hours with 50000 cpm/well of $^{125}$I-labelled polypeptide I (IL-1$\beta$) and 20 ng/ml of polypeptide I preincubated at 37°C in D-MEM supplemented with 10% FCS. The liquid reaction mixture was discarded with a Pasteur pipette, the cells were gently washed with 1 ml of D-MEM supplemented with 10% FCS, and the supernatant was discarded. After repeating the washing procedure twice, the cells were solubilized with 1 ml of a mixture of 1% SDS and 0.2 N NaOH. The radioactivity (bound radioactivity) of the solubilized cell solution and the liquid used for washing the wells was measured by a gamma-counter.

The $^{125}$I-labeled polypeptide I used was prepared and purified by the method of Bolton and Hunter (Biochem. J., 133, 529 (1973)). Specific activity: at least 250 uCi/µg protein. Table 7 below shows the results.

22

## Table 7

| Preincubation time (hr) | Inhibition activity (%) |
| --- | --- |
| 0 | 100 |
| 5 | About 40 |
| 30 | About 4 |

$$\text{Inhibition Activity (\%)} = \frac{A - B}{A - C} \times 100$$

wherein
- A: Bound radioactivity in absence of unlabelled polypeptide I
- B: Experimental value of bound radioactivity
- C: Radioactivity unspecifically absorbed on the plate

The foregoing index expresses the binding activity of the coexisting polypeptide I against IL-1 receptor.

Table 7 reveals that under the cytokine induction condition, the ability of polypeptide I, i.e. IL-1$\beta$ itself, to bind to the IL-1 receptor diminishes with time. Accordingly, the same testing procedure as above was repeated using polypeptide I, VI or XXXVII which was preincubated for 24 hours. Table 8 below shows the results.

**Claims**

1. Use of homogeneous IL-1$\beta$ for the preparation of a pharmaceutical composition for stimulating immunity.

2. Use of homogeneous IL-1$\beta$ for the preparation of a pharmaceutical composition for promoting production of CSF.

3. Use of homogeneous IL-1$\beta$ for the preparation of a pharmaceutical composition for curing inflammation.

23

# FIG. 1

# FIG. 2

EP 0 569 042 A1

FIG. 3

Fig. 4

24hr        48hr        72hr

CSF activity (units/ml)

Concentration of Polypeptide I

(GIF units/ml)

FIG. 5

FIG. 6

EP 0 569 042 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF CLINICAL IMMUNOLOGY vol. 5, no. 5, 1985, pages 287 - 297 C. A. DINARELLO ET AL 'An update on human interleukin-1: from molecular biology to clinical relevance' * the whole article, especially the abstract and page 293, right-hand column lines 14-46 * | 1-3 | A61K37/02 |
| P,X | IMMUNOBIOL. vol. 172, September 1986, pages 301 - 315 C. A. DINARELLO ET AL 'Multiple biological properties of recombinant human interleukin-1 beta' * the whole article , especially page 306, table 1 and page 301 line 1- page 302 line 20 * | 1-3 | |
| P,X | BIOCHEMICAL AND BIOPHYSICAL RESEARCH COMMUNICATIONS. vol. 141, no. 1, 26 November 1986, DULUTH, MINNESOTA US pages 285 - 291 K. SATO ET AL 'Recombinant human Interleukin -1 alpha and beta stimulate mouse osteoclast-like cells (MC3T3-E1) to produce macrophage-colony stimulating activity and prostaglandin E2' * abstract; figure 2B * * page 288 * | 1-3 | **TECHNICAL FIELDS SEARCHED (Int. Cl.4)** C07K A61K C12N |

-/--

The present search report has been drawn up for all claims

| Place of search | Date of completion of the search | Examiner |
|---|---|---|
| THE HAGUE | 26 JULY 1993 | LE CORNEC N.D.R. |

EPO FORM 1503 03.82 (P0401)

European Patent
Office

EUROPEAN SEARCH REPORT

Application Number

EP    93 10 7660
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | LYMPHOKINE RESARCH vol. 2, no. 3, 1983, MARY ANN LIEBERT INC.,PUBL. pages 97 - 102 R. F. KAMPSCHMIDT ET AL 'Infection, Inflammation and Interleukin-1(IL-1)' * the whole article , especially page 99, table 1 * | 1-3 | |
| A | REVIEWS OF INFECTIOUS DISEASES vol. 6, no. 1, 1984, pages 51 - 95 C. A. DINARELLO ET AL 'Interleukin -1' * page 66 - page 68 * * abstract * * page 73 - page 74 * * chapter V* * chapter IX * | 1-3 | |
| A | NATURE. vol. 315, no. 6021, 20 June 1985, LONDON GB pages 641 - 647 C. J. MARCH ET AL 'Cloning sequence and expression of two distinct' | | TECHNICAL FIELDS SEARCHED (Int. Cl.4) |
| E | WO-A-8 803 031 (IMMUNEX CORPORATION) * claims 1,5-8,11-12; figure 2; example 3 * | 3 | |

The present search report has been drawn up for all claims

| Place of search | Date of completion of the search | Examiner |
|---|---|---|
| THE HAGUE | 26 JULY 1993 | LE CORNEC N.D.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)